# EUROPEAN PATENT APPLICATION

(11) **EP 2 153 814 A1**
(43) Date of publication of application: **17.02.2010**
(21) Application number: 08013994.2
(22) Date of filing: 05.08.2008
(51) Int. Cl.: A61K 8/42, A61K 31/17, A61Q 17/00, A61Q 17/04, A61Q 19/00, A61Q 19/08

(54) **Use of compositions comprising urea**

(71) Applicant: ISDIN S.A., 08006 Barcelona (ES)
(72) Inventor: Trullas Cabanas, Carlos Ramon, 08232 Viladecavalls (ES); Krutmann, Jean Prof. Dr., 40225 Düsseldorf (DE)
(74) Representative: Brosch, Oliver

(57) **Abstract**

The present invention relates to the use of a composition comprising urea for increasing the epidermal expression of at least one gene selected from the group comprising transglutaminase-1, involucrin, filaggrin and loricrin, for preventing a decrease of the elasticity of the skin and for treating damages associated within.

## Description

The present invention relates to the use of a composition comprising urea for increasing the expression of epidermal markers. Furthermore the present invention relates to the prevention of various damages of the skin.

The human skin is a complex organ being subdivided into many different layers with each layer serving a specific purpose and thus expressing specific markers and comprising specific structures.
The outmost layers of the skin collectively constitute the epidermis. The layer underneath the epidermis is known as the dermis.

The epidermis is divided into 5 sublayers, which are present in the following order (from superficial to deep (bordering the dermis)): stratum corneum, stratum lucidum (only present at palms and soles), stratum granulosum, stratum spinosum and stratum germinativum (also known as stratum basale).
All layers are avascular and are supplied by the underlying dermis.
A prominent characteristic of the epidermis is that cells are born in the deep layers and migrate outward. Thus, cells which are present in the epidermis start their migration in the stratum germinativum.
The stratum germinativum largely consists of keratinocytes. These cells undergo rapid cell division to replenish the regular loss of skin by shedding from the surface. They then migrate through the stratum spinosum, a multi-layered arrangement of cuboidal cells. Cells in this layer often exhibit changes in their nuclear structure resulting from a shortage in nutrients. Another prominent characteristic of cells in this layer is the large number of intermediate filaments which are composed of keratin. The cells then reach the stratum granulosum, which is the outmost epidermal layer in which living cells are found. Cells found in the stratum granulosum contain many keratohyalin granules. Additionally these cells contain large amounts of filaggrin, a protein thought to serve in bundling keratin. Membrane-coated lamellar granules are also present and contain lipids. The lamellar granules are exocytosed in this layer to generate a waterproof barrier. This waterproofing also prevents life-sustaining nutrient transport, and thus leads to the characteristic cell death of the outer layers of keratinized epithelium.

The stratum corneum ("horny layer") is composed mainly of dead cells that lack nuclei. As these dead cells slough off, they are continuously replaced by new cells from the stratum germinativum (basale). The thickness of the stratum corneum varies according to the amount of protection and/or grip required by a region of the body. In general, the stratum corneum contains 15 to 20 layers of dead cells. The stratum corneum has a thickness between 10 and 40 µm. (=0.04mm).

Thus, several fundamental changes take place during the migration of the cells. One of these changes is the degeneration of the nucleus of keratinocytes. Furthermore, the differentiating cells change their morphology from a small and round shape to a larger size. Also, as already indicated above, large macromolecular networks start to assembly within the cell.
As these networks assemble, the so called cornified cell envelope structure is formed beneath the plasma membrane. It provides a vital physical barrier to these tissues in mammals and consists of a thick layer of highly cross-linked insoluble proteins.
In addition the corneocytes (the differentiated keratinocytes within the stratum corneum) start to accumulate a water soluble fraction of low molecular humectants which is termed natural moisturizing factor (NMF). NMF accounts for 15-20% of the total weight of the stratum corneum (Jacobi, 1959). NMF in general not only seems to be important for the water holding capacity of skin, but it also appears to increase the elasticity of the stratum corneum. Thus, if NMF is removed, water alone cannot restore elasticity.
These processes go along with changes in gene expression.
Genes encoding proteins being used for the formation of the fully keratinized epidermis, such as involucrin, loricrin, filaggrin and transglutaminase-1, start to be expressed in the stratum spinosum.
Involucrin, one of the earliest markers of terminal differentiation serves as a scaffolding component of the cornified envelope structure on to which other proteins bind.
Loricrin accounts according to its cysteine residues to the sulphur-rich contents of the keratohyalin granules.
Filaggrin is a cationic protein rich in histidine residues which is assumed to organize and aggregate keratin intermediate filaments into macrofibrils.

Transglutaminase-1 is a membrane-bound keratinocyte-specific transglutaminase which is largely responsible for the cross-linking of involucrin and loricrin to form the the cornified envelope.

These processes can be negatively affected by various intrinsic and extrinsic factors resulting in a reduced strength/level of the cornified cell envelope and the natural moisturizing factor respectively.

As indicated before, this reduction has several negative consequences. In particular, there is a loss in elasticity of the skin leading to the appearance of symptoms typically associated with aging.

The object of the present invention is to at least to prevent a decrease of the elasticity of the skin, preferably enhancing said elasticity.

As the degree of elasticity of the skin goes along with the integrity of epidermal structures such as the cornified cell envelope and the appearance of other large macromolecular networks which are being form during the differentiation of the keratinocytes, another object of the present invention is to provide a novel approach for increasing the level of proteins necessary for the formation of such structures.

Surprisingly, it was found that the topical application of compositions comprising defined concentrations of urea result in an increased expression of the genes transglutaminase-1, involucrin, filaggrin and loricrin which encode key proteins for the formation of the epidermal structures discussed above.

Accordingly, the present invention relates to the use of a composition comprising urea, preferably equal or more than 5% urea by weight, based on the total amount of the composition, for increasing the epidermal expression of at least one gene selected from the group comprising transglutaminase-1, involucrin, filaggrin and loricrin.

Preferably, the inventively used composition comprises at most 30% by weight, most preferably 10 to 25% by weight, based on the total amount of the composition, of urea.

The term "epidermal" or "epidermis" as used according to the present invention refers to the respective layer of mammals, especially of human beings.

The term "composition" as used according to the present invention comprises but is not limited to cosmetic and pharmaceutical compositions.

To achieve an optimal effect of the amount of urea present in the inventively used composition, the other components of said compositions need to be selected and composed accordingly.
Surprisingly, it became clear, that the effect of urea on the epidermis seems to be affected by the proportion of hydrophobic components in said compositions.

Thus, preferably, the compositions comprising urea comprises at least 50 % by weight, based on the total amount of the composition, of hydrophobic components.

One object of the present invention relates to the use of urea comprising compositions for increasing the expression of a gene according to the invention by a factor of at least 3, preferably at least 5, more preferably at least 7, even more preferably at least 9, most preferably at least 12.

As known to the person skilled in the art, various methods are available to determine the rate of expression of a gene.
Inventively said factor is determined by real time PCR as outlined in the section "Example".

The person skilled in the art will be able to specifically adapt the inventively used composition according to the intended application/administration to the epidermis. Exemplary formulations of application/administration are outlined further below.

The person skilled in the art will also be able to specifically adapt the composition according to the invention with respect to any regions of the human skin to which said composition should be administered.
Thus, the person skilled in the art will be able to select and/or avoid auxiliary substances which might be suitable or unsuitable for use in the different regions of the skin, e.g. eyes, face, neck, arms, legs etc.

Such a selection of components for an inventively used composition has also to take into account that such component does not infer with the effect of urea.
Subject to these provisions and depending on the specific requirements of the application, the inventively used compositions may contain one or more of the following of compounds: gelling agents, oils, waxes, thickening agents, hydrophilic or hydrophobic polymers, moisturizers, emulsifying agents, emollients, fatty acids, organic solvents, stabilizers, sequestering agents, acidifying or basifying agents, surfactants, film formers, biological additives to enhance performance and/or consumer appeal such as amino acids, proteins, vanilla, aloe extract or bioflavinoids, buffering agents, chelating agents such as ethylenediaminetetra-acetic acid (EDTA) or oxalic acid, colorants, dyes, propellants, antifoaming agents, wetting agents, vitamins, emulsion stabilizers, pH adjusters, thickening agents, fragrances, fillers, preservatives, opacifying agents, water and/or alcohols. The aforementioned auxiliary agents for the inventively used compositions are used in the usual amounts known by those skilled in the art.
As oils for the inventively used compositions oils from animal or vegetable sources or synthetic are preferably used. As oils can be used at least one oil selected from the group comprising mineral oil, liquid petrolatum, liquid paraffin, volatile and non-volatile silicone oils, isoparaffins, polyalphaolefins, fluorated and perfluorated oils. Examples of further suitable oils - depending on the compounds of the composition - comprise hydrocarbon-based oils of animal origin such as squalene, perhydrosqualene; hydrocarbon-based plant oils such as liquid triglycerides of fatty acids of 4 to 10 carbon atoms, for instance, heptanoic or octanoic acid triglycerides, or oils such as olive oil, macademia nut oil, sunflower oil, sunflower seed oil, corn oil, soybean oil, grapeseed oil, castor oil, avocado oil, caprylic/capric acid triglycerides, jojoba oil; linear or branched hydrocarbons of mineral or synthetic origin such as liquid paraffins and derivatives thereof, petroleum jelly; synthetic esters and ethers, in particular esters of fatty alcohols, like; for example, octyldodecyl myristate, glyceryl octanoate, ethylhexyl isononanoate,isopropyl myristate, 2-ethylhexyl palmitate, 2-octyldodecyl stearate, isostearyl isostearate; hydroxylated esters such as isostearyl lactate, octyl hydroxystearate, octyldodecyl hydroxystearate, heptanoates, octanoates and decanoates of fatty alcohols; polyol esters such as propylene glycol dioctanoate, neopentyl glycol diheptanoate, diethylene glycol diisononanoate, and pentaerythritol esters; fatty alcohols containing from 12 to 26 carbon atoms such as octyldodecanol, 2-butyloctanol, 2-hexyldecanol, 2-undecylpentadecanol, oleyl alcohol; partially hydrocarbon-based fluoro oils and/or fluorosilicone oils; silicone oils such as volatile or non-volatile, linear or cyclic polymethylsiloxanes (PDMS) that are liquid or semisolid at room temperature such as cyclomethicones and dimethicones, optionally comprising a phenyl group, for instance phenyl trimethicones, siloxanes, and mixtures thereof.
The oil phase of the inventively used compositions may comprise one or more waxes, gums, or mixtures thereof. The waxes include hydrocarbon-based waxes, fluoro waxes and/or silicone waxes and can be of plant, mineral, animal and/or synthetic origin.
The gums are e.g. high molecular weight PDMSs, cellulose gums or polysaccharides, and the semi-solid materials are generally hydrocarbon-based compounds, such as, but not limited to, lanolins and derivatives thereof, or alternatively PDMSs.

The term "moisturizer" as used according to the present invention means a compound that acts on the barrier function, in order to maintain the moisturization of the stratum corneum, or an occlusive compound. Mention may be made of ceramides, sphingoid-based compounds, lecithins, lycosphingolipids, phospholipids, cholesterol and derivatives thereof, phytosterols (stigmasterol, β-sitosterol or campesterol), essential fatty acids, 1,2-diacylglycerol, 4-chromanone, pentacyclic triterpenes such as ursolic acid, liquid petroleum jelly and lanolin; or a compound that directly increases the water content of the stratum corneum, such as threalose and derivatives thereof, hyaluronic acid and derivatives thereof, glycerol, pentanediol, sodium pidolate, serine, xylitol, sodium lactate, polyglyceryl acrylate, ectoin and derivatives thereof, chitosan, oligosaccharides and polysaccharides, cyclic carbonates, N-lauroylpyrrolidonecarboxylic acid and N-α-benzoyl-L-arginine; or a compound that activates the sebaceous glands, such as DHEA, 7-oxide and/or 17-alkyl derivatives thereof, sapogenins and vitamin D and derivatives thereof.
As stabilizers for the inventively used compositions preferably non-ionic, anionic, cationic and amphiphilic tensides can be used, which are preferably selected from the group comprising polyethylenglycol (PEG) and derivatives thereof, tweens, tritons, spans, polyglycerines, polyalkyl glycerides, alkyl sulfonates, aryl sulfonates, alkyl phosphates, derivatives of alkyl-betaine and phosphatidylglycerole.
Emulsifiers are preferably used in certain formulations of the inventively used compositions in amounts effective to provide uniform blending of ingredients of the composition. Useful emulsifiers include anionics such as fatty acid soaps, e.g., potassium stearate, sodium stearate, ammonium stearate, and triethanolamine stearate; polyol fatty acid monoesters containing fatty acid soaps, e.g., glycerol monostearate containing either potassium or sodium salt; sulfuric esters (sodium salts), e.g., sodium lauryl 5 sulfate, and sodium acetyl sulfate; and polyol fatty acid monoesters containing sulfuric esters, e.g., glyceryl monostearate containing sodium lauryl surfate; (ii) cationics chloride such as N(stearoyl colamino formylmethyl) pyridium; N-soya-N-ethyl morpholinium ethosulfate; alkyl dimethyl benzyl ammonium chloride; diisobutylphenoxytheoxyethyl dimethyl benzyl ammonium chloride; and acetyl pyridium chloride; and (iii) nonionics such as polyoxyethylene fatty alcohol ethers, e.g., monostearate; polyoxyethylene lauryl alcohol; polyoxypropylene fatty alcohol ethers, e.g., propoxylated oleyl alcohol; polyoxyethylene fatty acid esters, e.g., polyoxyethylene stearate; polyoxyethylene sorbitan fatty acid esters, e.g., polyoxyethylene sorbitan monostearate; sorbitan fatty acid esters, e.g., sorbitan; polyoxyethylene glycol fatty acid esters, e.g., polyoxyethylene glycol monostearate; and polyol fatty acid esters, e.g., glyceryl monostearate. Preferred emulsifiers are glycerol stearate, PEG-40-stearate, sodium carbomer, acrylate/C10-C30-alkyl acrylate crosspolymer.
Surfactants can be used in certain formulations of the inventively used compositions. Suitable surfactants are for example those surfactants generally grouped as cleansing agents, emulsifying agents, foam boosters, hydrotropes, solubilizing agents, suspending agents and non-surfactants, which facilitate the dispersion of solids in liquids. Examples of the surfactants include sorbitan sesquioleate, polysorbate 60, glyceryl stearate, lipophilic glyceryl stearate, sorbitan oleate, sorbitan stearate, DEA-cetyl phosphate, sorbitan stearate/sucrose cocoate, glyceryl stearate/polyethylene glycol- 100 stearate, ceteareth-6 olivate, arachidyl alcohol/behenyl alcohol/arachidyl glucoside, polypropylene glycol- 26-buteth-26/polyethylene glycol-40, and hydrogenated castor oil. Glycerin, sorbitol, propylene glycol, butylene glycol, hexylene glycol, diglycerin, betaine, glycereth-26, or methylgluceth-20 can be used as humectants. Cetyl alcohol, stearyl alcohol, octyl dodecanol, and isostearyl alcohol can be used as higher alcohols.

Xanthan Gum, carbomer, magnesium aluminium silicate, cellulose gum, and/or dextrin palmitate can be used as thickeners. Disodium EDTA and tetrasodium EDTA can be used as chelating agents. Blue No. 1, Red No. 40, and Yellow No. 4 and 5 can be used as colorants.
Suitable film formers which are preferably used in the formulations of the inventively used compositions should keep the composition smooth and even and are preferably, without limitation, at least one substance selected from the group comprising acrylamide/sodium acrylate copolymer; ammonium acrylates copolymer;
Balsam Peru; cellulose gum; ethylene/maleic anhydride copolymer; hydroxyethylcellulose; hydroxypropylcellulose; polyacrylamide; polyethylene; polyvinyl alcohol; pvm/MA copolymer (vinyl methylether/maleic acid anhydride copolymer); PVP (polyvinylpyrrolidone); maleic acid anhydride polymer, vinylpyrrolidon/hexadecene copolymer; acrylate copolymer and the like.
The person skilled in the art will be able to adjust the pH-value of the inventively used composition to a suitable pH-value for the particular application.

pH adjusters may also be used in certain formulations of the inventively used compositions. These pH adjusters preferably comprise, but are not limited to ammonium hydroxide, triethanolamine or citric acid.

As further thickening agents used for the inventively used compositions can preferably be used candelilla, carnauba, and microcrystalline waxes, crosslinked acrylic-acid polymers, carbomer, methylhydroxyethylcellulose, hydroxypropylmethylcellulose or hydroxyethylcellulose. and polyethylene thickeners.

Gelling agents, which can be preferably used in the formulations of the inventively used compositions, can be natural or synthetic polymers. Natural polymers are preferably selected from the group comprising Agar-Agar, alginate, pectin, carbomer, carrageenan, casein, dextrine, gelatine, arabic gum, keratine, locust bean gum, xanthan gum and the like. Preferred synthetic polymers, which can be used in the formulations of the inventively used compositions are selected from the group comprising acylic acid polymers, polyacryl amides and alkylene oxide polymers.

Exemplary fillers include but are not limited to talc, silica, zinc stearate, mica, kaolin, nylon (in particular orgasol) powder, polyethylene powder, Teflon, starch, boron nitride, microspheres of copolymers such as Expancel (Nobel Industrie), Polytrap (Dow Coming), and silicone resin microbeads (Tospearl from Toshiba).

The inventively used compositions may also include e.g. a skin penetration enhancer, a skin plumber and/or an optical diffuser.
As skin plumpers a collagen enhancer to the skin can serve. An example of a suitable and preferred skin plumper is palmitoyl oligopeptide. Other skin plumpers are collagen and/or glycosaminoglycan (GAG) enhancing agents. The skin plumper can be preferably present in an amount of from about 0.1 wt % to about 20 weight %, based on the total weight of the composition.
An optical diffuser comprises particles that change the surface optometrics of skin, resulting in a visual blurring and softening of, for example, lines and wrinkles. Examples of such optical diffusers that can be used in the inventively used compositions include, but are not limited to, boron nitride, mica, nylon, polymethylmethacrylate (PMMA), polyurethane powder, sericite, silica, silicone powder, talc, Teflon, titanium dioxide, zinc oxide, or any mixtures thereof. An optical diffuser is preferably present in an amount of from about 0.01 weight % to about 20 weight %, based on the total weight of the composition.

The inventively used composition may also include nutritionally desirable minerals.

Other additives include beneficial agents such as those materials that condition.

Other additives include beneficial agents such as those materials that condition the skin (particularly, the upper layers of the skin in the stratum corneum) and keep it soft by retarding the decrease of its water content and/or protect the skin. Such conditioners and moisturizing agents include, by way of example, pyrrolidine carboxylic acid and amino acids; other additives comprise cosmetically and/or cosmeceutically active agents. Such additional cosmetically and/or cosmeceutically active agents include, for example, alpha hydroxyacids, alpha ketoacids, polymeric hydroxyacids, moisturizers, collagen, marine extract.

Other agents may also be added, such as antimicrobial agents, to prevent spoilage upon storage, i.e., to inhibit growth of microbes such as yeasts and molds. Exemplary organic antimicrobrial agents are e.g. 2,4,4'-trichloro-2-hydroxy diphenyl ether (triclosan) and benzoic acid.
The compositions may also contain irritation-mitigating additives to minimize or eliminate the possibility of skin irritation or skin damage resulting from the chemical entity to be administered, or other components of the composition. Exemplary irritation-mitigating additives include, for example: α-tocopherol; monoamine oxidase inhibitors, particularly phenyl alcohols such as 2-phenyl-1-ethanol; glycerin; salicylates; ascorbates; ionophores such as monensin; amphiphilic amines; ammonium chloride; N-acetylcysteine; capsaicin; and chloroquine.

The compositions may also contain anti-inflammatory agents. Exemplary anti-inflammatory agents are e.g. acetylsalicylic acid and glycyrrhetinic acid.

The compositions may also contain anti-seborrhoeic agents, vasodilators, inhibitors of melanogenesis, anti-allergenics, antifungals, antiseptics, , analgesics, nitric oxide synthase inhibitors, insect repellents, self-tanning agents, skin penetration enhancers, skin cooling agents, chelating agents, colorants including dyes, lakes and pigments that may be untreated or chemically surface treated to improve wetability or some other property, pearlescent agents, demulcents, agents for stimulating the synthesis of dermal or epidermal macromolecules and/or for preventing their degradation; agents for stimulating fibroblast proliferation and/or for stimulating keratinocyte differentiation; muscle relaxants; tensioning agents; antipollution agents and/or free-radical scavengers, sunscreens and mixtures thereof, anesthetics, antineoplastics, antivirals, hypopigmenting agents, immune system boosting agents, immune system suppressing agents, insect repellents, lubricants, matting agents, photostabilizing agents, preservatives, skin protectants, skin penetration enhancers, staining agents, sunscreens, viscosity and/or rheology modifiers.

The pigments which may be added to the inventively used compositions comprise inorganic pigments and organic pigments. Suitable inorganic pigments include, but are not limited to, titanium oxide, zirconium oxide and cerium oxide, as well as zinc oxide, iron oxide, chromium oxide and ferric blue. Suitable organic pigments include, but barium, strontium, calcium, and aluminium lakes and carbon black.

Suitable pearlescent agents include mica coated with titanium oxide, with iron oxide, or with natural pigment.

The depigmenting agents that may be incorporated into the composition according to the present invention comprise, for example, the following compounds: kojic acid; ellagic acid; arbutin and derivatives thereof such as those described in U.S. Pat. No. 6,306,376 and U.S. Pat. No. 5,346,693; hydroquinone, without this list being limiting. The composition according to the invention may comprise dermo-decontracting agents, among which mention may be made in particular of alverine and salts thereof, especially alverine citrate, sapogenins such as diosgenin and natural extracts containing it (such as extracts of wild yam), certain secondary and tertiary carbonyl amines, organic or mineral salts of metals, in particular manganese gluconate, adenosine, and also the hexapeptide argireline R sold by the company Lipotec. Mention may also be made of certain fragrancing compositions with a dermo-decontracting effect.

As far as the invention relates to a cosmetic composition, it is further referred to International Cosmetic Ingredient Dictionary and Handbook (ICID), 10th Edition (2004), published by the Cosmetic, Toiletry, and Fragrance Association (CTFA), at pp. 2177-2299, which includes information about suitable cosmetic ingredients and their use and which is herein incorporated by reference in its entirety.

For the inventively used cosmetic composition marketed in Europe the respective components shall be used in amounts which are in compliance with the Council Directives 76/768/EEC and Commission Directive 95/17/EC on the approximation of the laws of the Member States relating to cosmetic products.

As it will be obvious to the person skilled in the art, the composition may contain many more compounds suitable for the specific requirement. Some further categories of compounds are disclosed herein after.

Depending on the specific constitution of any of the inventively used compositions, the person skilled in the art will be able to select the appropriate steps for preparing said composition.

The particular combination of components in the composition is determined largely by chemical compatibility. The person skilled in the art will be able to easily determine which of the compounds listed above and which alternative compounds known to the person skilled in the art might be used/combined for a specific composition according to the invention.

Furthermore the particular combination of components in the composition will depend on the type of application, the specific purpose being addressed by the composition and the like. The inventively used composition may also be specifically adapted to the needs of a specific region of the skin.

The compositions comprising urea are preferably topically applied onto the skin or administered by injection.

For the preferred topical application, the inventively used composition may be formulated in liquid or in semi-solid form, preferably as liquid, fluid, foam, cream, gel, paste, balsam, spray, ointment, lotion, conditioner, tonic, milk, mousse, emulsion, serum, oil, stick, shampoo, jelly, suspension, dispersion, lacquer, paint, elixir, drop, aerosol, day creams or lotions, night creams or lotions, salves, sunscreen creams, fluid lotions, oils, water-in-oil, oil-in-water, water-oil-water triple emulsions, masks, body milks, makeup, artificial tanning compositions, depilatories, emulsifiers, patches, or a solid which is poured or cast as a stick or a dish, microemulsions, aerosol, powder, soap, surfactant-containing cleansing powder foundation, emulsion foundation, wax foundation, softening lotion, nutrient lotion, nutrient cream, massage cream, bath compositions, aftershave gels or lotions, cleansing cream, cleansing foam, cleansing water or packs, composition for countering insect stings or bits, pain-relieving compositions or compositions for treating certain skin diseases or skin disorders. It is also contemplated that topical compositions of the present invention can be incorporated into delivery systems such as liposomes, nano particles, topical patches, tapes and sprays. It may also be formulated in a makeup product for facial skin, the body or the lips, such as a foundation, a tinted cream, a makeup rouge, an eyeshadow, a loose or compact powder, a concealer stick, a cover stick, a lipstick or a lipcare product.

The compositions containing urea can also be injected into the skin that should be treated.

For such an injection, the composition can be provided in the form of aqueous or oily lotions or in the form of serums.

Preferably, the pH-value of the inventively used composition will be adjusted using a suitable puffering agent. Preferably the pH-value of the inventively used composition will be in the range of 3,5 - 9,0 for topical application.

For injection, the preferred pH-value of the inventively used composition will be in the range of 3,0 - 9,0.

The composition used according to the invention might be applied to the complete skin surface or at least significant parts thereof. The application might also be limited to specific regions of the body which are particularly vulnerable for factors triggering processes resulting in a decrease of elasticity. Regions which might be very vulnerable to photoaging are those regions that are typically exposed to sunlight such as the face, ears, bald areas of the scalp, neck, décolleté, lips, forearms, wrists, hands and feet.

Alternatively, the administration might be limited to regions of the skin which are particularly likely to exhibit any damages according to the invention.

For a successful treatment of the skin the compositions comprising urea is applied onto the skin at least once a day for at least 2 weeks topically and optionally longer until the expression of any of said genes is increased by a factor of at least at least 3, preferably at least 5, more preferably at least 7, even more preferably at least 9, most preferably at least 12.

A successful treatment of the skin can also be achieved by injections, preferably by, optionally repeated, injections of a depot.

In total the period of application and/or administration can continue for several weeks or even several months or even several years either continuously or in repeated phases of application.

In general the method of delivery of the active agent may vary, but necessarily involves application of the inventively used composition to the area of the skin were the desired effect is intended.

As indicated before the application and/or administration of the inventively used composition affects the degree of elasticity of the skin in the treated region.

Thus, one further embodiment of the present invention relates to a use of a urea-containing composition for at least preventing damages of the skin preferably preventing a decrease of elasticity of the skin, and/or for enhancing said elasticity.

The term "preventing a decrease in elasticity" as used according to the present invention refers to the stop or the slow down of the decrease in elasticity in comparison to the elasticity at the time at which the treatment according to the invention starts.

Similarly the term "enhancing" as used according to the present invention, and as far as said term relates to the elasticity of the skin, refers to an increase of the elasticity based on the degree of elasticity at the point in time at which the treatment according to the invention commences.

As the compositions according to the invention affect the expression of proteins which are necessary for keeping the skin moisturized and sufficiently elastic, there is clearly a cosmetic application.

One further embodiment of the present invention relates to the use of a composition comprising urea adjusted as a cosmetic composition.

Such a cosmetic composition may also include other components which are useful for moisturizing the skin and/or preventing and/or treating a decreased elasticity of the skin. In general, such a cosmetic composition may include other components which are useful for preventing signs of aging.

Preferably said cosmetic composition comprises optionally at least one compound selected from the group comprising UV-filters, vitamins, coenzymes, retinoids, phytokines and antioxidative agents.

Suitable vitamins are for example vitamin A, vitamins of the vitamin B group, e.g. vitamin B1 and B5, vitamin C, vitamins of the vitamin D group such as for example vitamin D and vitamin D3, and vitamin E. As it will be obvious to the person skilled in the art, the inventively used compositions may (also) comprise suitable derivatives of said vitamins such as the vitamin D-derivative 1-fluoro-25-hydroxy-16-ene-23-yne-hexa:fluoro-cholecalciferol.

An antioxidant functions, among other things, to scavenge free radicals from skin to protect the skin from environmental aggressors. Examples of antioxidants that may be used in the present compositions include compounds having phenolic hydroxy functions, such as ascorbic acid and its derivatives/esters e.g. sodium-L-ascorbate, calcium-L-ascorbate, ascorbyl palmitate; beta-carotene; catechins; curcumin; ferulic acid derivatives (e.g. ethyl ferulate, sodium ferulate); gallic acid derivatives (e.g. propyl gallate); lycopene; reductic acid; rosmarinic acid; tannic acid; tetrahydrocurcumin; tocopherol and its derivatives e.g. α-tocopherol, γ-tocopherol, δ-tocopherol; uric acid; or any mixtures thereof. Also propylgallate, octylgallate, dodecylgallate, sodium-isoascorbate, citric acid, sodium citrate, potassium citrate and tin-II-chloride. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur.

Retinoids which can be used additionally include, without limitation, retinoic acid (e.g., all-trans or 13-cis) and derivatives thereof, retinol (Vitamin A) and esters thereof, such as retinol palmitate, retinol acetate and retinol propionate, and salts thereof.

In addition, it is also possible to include known agents which stimulate proliferation of keratinocytes and can be formulated with the compositions used according to the invention.

Other known components which are suitable for preventing and/or treating signs of aging might be included in the inventively used composition.

Additionally/Alternatively compounds can be included into the compositions according to the invention which are known to stimulate the macromolecules of the dermis or prevent their degradation influencing the synthesis of collagen, such as extracts of Centella asiatica; asiaticosides and derivatives; ascorbic acid or vitamin C and its derivatives, such as ascorbyl glucoside (marketed by Hayashibara); synthetic peptides, such as iamine, the palmitoyl of glycine-histidine-lysine tripeptide, marketed under the name "Biopeptide CL" by Sederma; peptides extracted from plants, such as the soybean hydrolysate marketed by Coletica under the trademark Phytokine®; extracts of soya fibers, such as that marketed under the name "Raffermine" by Silab; plant hormones, such as auxins and lignans; the palmitoyl of lysine-threonine-threonine-lysine-serine pentapeptide marketed in particular under the name "Matrixyl" by Sederma; dimethylaminoethanol; extracts of Bupleurum chinensis rhizome, such as those marketed under the names "Pleurimincyl" or "Lipocare" by Sederma; acylated with a hydrolysates of wheat protein, in particular acylated with a palmitoyl group, such as that marketed under the name "Lipacid PVB" by Seppic; creatine; coenzyme Q10; retinol; dipalmitoyl hydroxyproline, in particular marketed by Seppic under the name "Sepilift DPHP", or extracts of red clover (Trifolium pretense) comprising isoflavones; or the synthesis of elastin, such as the extract of Saccharomyces cerivisiae marketed by LSN under the trademark Cytovitin®; and the extract of the alga Macrocystis pyrifera marketed by Secma under the trademark Kelpadelie®; or the synthesis of glycosaminoglycans, such as the product of fermentation of milk by Lactobacillus vulgaris marketed by Brooks under the trademark Biomin yogourth®; the extract of the brown alga Padina pavonica marketed by Alban Müller under the trademark HSP3®; and the extract of Saccharomyces cerevisiae available in particular from Silab under the trademark Firmalift® or from LSN under the trademark Cytovitin®; or the synthesis of fibronectin, such as the extract of Salina zooplankton marketed by Seporga under the trademark GP4G®; the yeast extract available in particular from Alban Müller under the trademark Drieline®; and the palmitoyl pentapeptide marketed by Sederma under the trademark Matrixil®; or the synthesis of compounds present at the dermal-epidermal junction (such as collagen VII and/or collagen IV) and/or laminin, such as dipalmitoyl hydroxyproline, marketed in particular by Seppic under the name "Seppilift DPHP", or phytosterol sulfate, such as that marketed by Vincience under the name "Phytocohesine"; or the inhibition of metalloproteinases (matrix metalloproteinases or MMPs), such as more particularly MMP 1, 2, 3 or 9. Mention may be made of retinoids and derivatives, oligopeptides and lipopeptides, lipoamino acids, the malt extract marketed by Coletica under the trademark Collalift®; extracts of blueberry or of rosemary; lycopene; isoflavones, their derivatives or the plant extracts comprising them, in particular extracts of soybean (marketed, for example, by lchimaru Pharcos under the trademark Flavosterone SB®), of red clover (marketed, for example, by Sederma under the trademark "Sterocare®"), of flax, of kakkon or of sage; extracts of Curcuma longa; or Siegesbeckia extracts (marketed, for example, by Sederma); or on the inhibition of serine proteases, such as leukocyte elastase or cathepsin G. Mention may be made of the peptide extract of leguminous plant (Pisum sativum) seeds marketed by LSN under the trademark Parelastyl®; heparinoids; and pseudodipeptides, such as {2-[acetyl(3-(trifluoromethyl)phenyl)amino]-3-methylbutyrylamino}acetic acid.

Further known compounds which affect the collagen synthesis in the dermis (and are thus suitable for preventing signs of aging triggered by/associated with a degradation of the collagen matrix in the dermis) can be added into the inventively used compositions which for example are compounds disclosed in the following publications already mentioned before: WO07094533 ("Composition for inhibiting collagen degradation and promoting collagen synthesis comprising emodin"), WO07075016 ("Collagenase inhibitor containing poly-gamma-glutamic acid vitamin C complex and use thereof"), EP0809484 ("Cosmetic or pharmaceutical, particularly dermatological, composition containing a bertholletia extract" - disclosing a bertholletia extract), EP1566177 ("The use of bisphosphonates for treating skin by stimulating collagen synthesis" - disclosing certain bisphosphonates), WO06083087 ("Method for stimulating collagen synthesis and/or KGF expression" - disclosing an AIMP1-fragment), W007094533 ("Composition for inhibiting collagen degradation and promoting collagen synthesis comprising emodin" - disclosing emodin), WO05/037270 ("Composition for promoting synthesis of collagen, and composition for external preparation for skin comprising the same"), WO03055444A2 ("Resveratol analogues"), EP1889642A2 ("Cosmetic compositions combining a C-glycoside derivative and a N-acylaminoamide derivative"), EP1582208A3 ("An amino acid compositions for promoting collagen synthesis" - disclosing certain amino acid compositions), WO03078470A1 ("Fusion peptide grafted with TAT-peptide to human type-I collagen derived peptide, its preparation method and cosmetic composition for anti-aging comprising the same" - disclosing certain fusion peptides, JP08231370A2 " Skin cosmetic", JP08208424A2 "Cosmetic composition containing betulinic acid", WO9951220 "Methods and compositions for reducing UV-induced inhibition of collagen synthesis in human skin" or described e.g. in (G. Bellon et al. (1995) Biochimica Biophysica Acta, 1268, 311-323) or Cardinale G et al. (1974) Adv. Enzymol 41: 425.

The disclosure of the relevant compounds in these publications shall be regarded as disclosure of the present application or by incorporation of the relevant parts.

Other known substances which can be added to the inventively used compositions promote the synthesis of collagen are TGF (see Cardinale G et al. (1974) Adv. Enzymol 41: 425), a protein originated from animal placenta (JP08231370A2 " Skin cosmetic"), betulinic acid (JP08208424A2 "Cosmetic composition containing betulinic acid") which relevant disclosure is incorporated by reference herein.
Similarly, compounds have been developed for reducing UV-induced inhibition of collagen synthesis thereby stimulating collagen synthesis (see e.g. the compounds as disclosed in WO9951220 "Methods and compositions for reducing UV-induced inhibition of collagen synthesis in human skin").

As known components which stimulate the proliferation of fibroblasts within the skin and can be formulated with the composition used according to the invention are for example selected from plant proteins and polypeptides, extracts, in particular of soybean (for example, a soybean extract marketed by LSN under the name Eleseryl SH-VEG 8® or marketed by Silab under the trademark Raffermine®; and plant hormones, such as gibberellins and cytokinins.

The disclosure of such compounds as one component of the inventively used composition is incorporated by reference herein.

Furthermore, the compositions used according to the present invention may include sunscreens for preventing signs of aging, especially loss of elasticity of the skin additionally.

Such sunscreens could be particulate materials, among them are zinc oxide, titanium oxide, clays and ferric chloride. Because such material might be visible and occlusive, many people consider the corresponding opaque formulations cosmetically unacceptable. Other sunscreens rely on chemicals such as p-aminobenzoic acid (PABA), oxybenzone, dioxybenzone, ethylhexyl-methoxy cinnamate, octocrylene, octyl methoxycinnamate, and butylmethoxydibenzoylmethane that are transparent or translucent on the skin. While these materials may be more acceptable cosmetically, they are still relatively short-lived and susceptible to being removed by washing or perspiration.

Therefore improved UV-filters have been developed which can be used as auxiliary compounds like the UV-filters preventing/treating signs of aging of the skin disclosed e.g. in: WO07025599A1 "sunscreen composition comprising a dibenzoylmethane, an aminohydroxybenzophenone, a triazine and a triazole as UV filters"; WO07002047A1 "a product release system to atomize cosmetic hair or skin compositions containing UV filters"; WO06032741A1 "silane merocyanine sulphone derivatives; photoprotecting compositions containing same; use thereof as UV filter"; W005094772A3 "use of benzophenone UV filters for preventing tanning"; W005094772A2 "use of UV filters for preventing tanning"; WO04082580A3" photostabilized topical formulations of ketoprofen containing two UV filters"; WO04022015A1 "use of diketopiperazine derivatives as photostable UV-filters in cosmetic and pharmaceutical preparations"; W003039447A3 "composition containing an amino acid n-acylated ester and a polyamide-structured UV filter"; W003039447A2 "composition containing an amino acid n-acylated ester and a polyamide-structured UV filter"; WO0239972A1 "insoluble organic UV filters and cosmetic use thereof"; WO0149686A1 "s-triazine derivatives and their use as UV filters"; W00126618A3 "composition, especially a cosmetic composition, containing a steroid and a liposoluble UV filter"; W00126618A2 "composition, especially a cosmetic composition, containing a steroid and a liposoluble UV filter"; WO9825922A1 "insoluble s-triazine derivatives and their use as UV filters".
The disclosed UV-filters in these publications are also incorporated by reference herein as possible components in the urea-containing compositions.

Preferably as one component in the urea-containing compositions the UV-filters is a benzotriazolylphenole or a derivative thereof, preferably a silylated derivative, capable of filtering UV-light and/or a pyrroltriazine or a derivative thereof capable of filtering UV-light and/or a heptaazaphenalene or a derivative thereof capable of filtering UV-light.

Such UV-filters are disclosed in the documents PCT/2008/003356 "Compuestos de benzotriazlilfenol sililados sustituidos" and in W02006/128791 "New derivatives of pyrrolyltriazine together with methods for obtaining them and their use as protecting agents against UV radiation" and in W02007/006807 "New derivatives of heptaazaphenalene, methods for obtaining them, and theirs use as protecting agents against UV radiation" respectively which are incorporated by reference herein.

All these auxiliary components optionally used for formulating the urea-containing compositions have to fulfill the requirement not to interfere with the effect of urea. The same applies to all other components listed herein as well as of any combination thereof.

A decrease in the elasticity of the skin is often associated with other damages to the skin. Thus, preventing a decrease in elasticity or enhancing said elasticity will in general be advantageous for preventing and/or treating of damages of the skin associated with a decrease of elasticity.

Thus, the present invention also relates to the use of an urea-containing composition for preventing and/or treating damages of the skin associated with a decrease of elasticity.

The term "preventing damages" as used according to the present invention refers to the avoidance or to the slow down of the occurrence of damages of the skin.

The term "treating damages" as used according to the present invention refers to weakening the severity and/or elimination of damages of the skin.

The term "damages" as used according to the present invention refers to any damages of the skin, either caused by intrinsic and/or extrinsic factors especially associated with aging and/or exposure to at least one environmental toxins and/or UV-light and/or dermatoses and/or infections of the skin and especially related to a decrease of elasticity.

Thus, according to the present invention the urea-containing compositions are useful to prevent and/or treat damages which are associated with aging and/or exposure to at least one environmental toxins and/or UV-light and/or with dermatoses and/or infections of the skin.

Processes associated with aging are caused by intrinsic as well as extrinsic factors. Intrinsic aging, also known as the natural aging process, is a continuous process that normally begins in the mid-20s of human beings. Within the skin, collagen production slows, and the elastin-structures become less flexible. Dead skin cells do not shed as quickly and turnover of new skin cells may decrease slightly. While these changes usually begin in the 20s, the signs of intrinsic aging are typically not visible for decades.

Damages associated with aging include signs of aging which may include e.g. fine wrinkles, thin and transparent skin, loss of underlying fat which leads to hollowed cheeks and eye sockets as well as noticeable loss of firmness on the hands and neck, shrinking away of bones from the skin due to bone loss causing sagging skin, dry skin, inability to sweat sufficiently, graying hair, thinning of the nail plate, skin atrophy appearance and/or depth of lines and/or wrinkles, including fine lines, skin discoloration, including dark eye circles, skin sagging, skin fatigue and/or stress, e.g. skin breakout, skin flakiness, cellular aging, loss of skin tone, elasticity and/or luster, loss of skin firmness, poor skin texture, loss of skin elasticity and/or resilences.

An important intrinsic factor facilitating signs of aging on the skin are hormonal changes e.g. during menopause.
A number of extrinsic, or external, factors often act together with the normal aging process to age the skin prematurely. Most premature aging is caused by sun exposure. Other external factors that age the skin prematurely are repetitive facial expressions, gravity, sleeping positions, temperature, humidity, environmental pollution, irritants (detergents, acids, bases, oxidizing agents, reducing agents, concentrated solvents, toxic gases or fumes), mechanical stresses (friction, impacts, abrasion, tearing of the surface, projection of dusts or particles, shaving or hair removal), thermal or climatic imbalances (cold, dryness or radiation), xenobiotics (undesirable microorganisms, allergens) and smoking.
Also, e.g. chronobiological stress accelerates aging thereby also affecting the skin. Sun exposure leads to photoaging. The skin loses the ability to repair itself, and the damage accumulates. Repeated ultraviolet (UV) exposure breaks down collagen fibers and impairs the synthesis of new collagen. The sun also attacks elastin. Sunweakened skin ceases to be resilent much earlier than skin protected against UV rays. Skin also becomes loose, wrinkled, and leathery much earlier because of unprotected exposure to sunlight.

Some of the signs of aging mentioned above are in particularly prominent in photoaging. The photoaged skin can often be the thicker than normal (acanthotic) and then atrophies over time. Thus photoaging is in particular characterized clinically by coarseness, wrinkles, mottled pigmentation, sallowness, laxity, telangiectasia, lentigines, purpura and relative ease of bruising, atrophy, depigmented areas, eventually premalignant, and ultimately malignant neoplasms.

Infections of the skin may be caused by either fungi, yeast, bacteria or any combination thereof.

As the genes according to the invention (transglutaminase-1, involucrin, loricrin, filaggrin) are associated with cellular processes resulting in the formation of a cornified cell envelope and other macromolecular structures, up-regulation of their expression is not only advantageous for cosmetic applications, but may also be beneficial in pharmaceutical therapy. This is achieved because a skin which is in "good condition" will in generally be more resistant to various diseases and disorders as well as because facilitating the differentiation processes in the epidermis may be beneficial for achieving physiological conditions.

Thus, the present invention also refers to the use of a composition comprising urea and optionally at least one pharmaceutically active agent as further component.

Such component can be at least one pharmaceutically active agent selected from the group comprising antibiotics, anti-infectives, pharmaceutically active agents against dermatoses and active agents for wound healing.

Such pharmaceutically active agents are well known in the field.

They include but are not limited to antimicrobial agents; antibacterial agents; antipruritic and antixerotic agents; antiinflammatory agents; local anesthetics and analgesics; corticosteroids; retinoids; vitamins; hormones; and antimetabolites and are at least one active agent selected from the group comprising acyclovir, amphotericins, chlorhexidine, clotrimazole, ketoconazole, econazole, miconazole, metronidazole, minocycline, nystatin, neomycin, kanamycin, phenytoin, pare-amino benzoic acid esters, octyl methoxycinnamate, octyl salicylate, oxybenzone, dioxybenzone, tocopherol, tocopheryl acetate, selenium sulfide, zinc pyrithione, diphenhydramine, pramoxine, lidocaine, procaine, erythromycin, tekacycline' clindamycin, crotamiton, hydroquinone and its monomethyl and benzyl ethers, naproxen, ibuprofen, cromolyn, retinol, retinyl palmitate, retinyl acetate, coal tar, griseofulvin, estradiol, hydrocortisone, hydrocortisone 21-acetate, hydrocortisone 17-valerate, hydrocortisone 17-butyrate, progesterone, betamethasone valerate, betamethasone dipropionate, triamcinolone -20 acetonide, fluocinonide, clobetasol propionate, minoxidil, dipyridamole, diphenylhydantoin, benzoyl peroxide, and 5-fluorouracil.

A pharmacological acceptable carrier may also be incorporated in the inventively used composition and may be any carrier conventionally used in the art.

Additional examples of pharmaceutically active agents that can be used as components in the inventively used compositions can be found e.g. in "Rote Liste - Arzneimittelverzeichnis für Deutschland" (Edition 2008, published by Rote Liste Service GmbH, Frankfurt/Main, Germany) which is incorporated by reference herein. Additional examples of pharmaceutically active agents for use in dermatology can be found in e.g. "Hauptgruppe 32 'Dermatika'" of the Rote Liste (Edition 2008) which are incorporated by reference herein.
As discussed before, examples of antibiotics, anti-infectives and pharmaceutically active agents against dermatoses are well known in the field.
Examples of such pharmaceutically active agents can also be found e.g. in the Rote Liste which is, as outlined before, incorporated by reference herein.
Specifically, examples of antibiotics can be found e.g. in "Hauptgruppe 10 'Antibiotika/Antiinfektiva'" of the Rote Liste.
Examples of anti-infectives can be found e.g. in "Hauptgruppe 10 'Antibiotika/Antiinfektiva' as well as "Hauptgruppe 21 'Antimykotika'" of the Rote Liste.
Examples of pharmaceutically active agents against dermatoses can be found e.g. in "Hauptgruppe 32 'Dermatika'" of the Rote Liste.

Examples of pharmaceutically active agents for wound healing can be found e.g. in "Hauptgruppe 32 'Dermatika'" as well as "Hauptgruppe 85 'Wund- und Narbenbehandlungsmittel'" of the Rote Liste.
The person skilled in the art will be able to select the appropriate pharmaceutically active agent according to the information presented therein and his general knowledge in the field.
Depending on the specific pharmaceutically active agent, the person skilled in the art will be able to adapt the specific composition accordingly either by obvious preliminary tests or by the general knowledge in the field.

### Example

### Compositions

Emulsions were used as listed thereafter.

### Emulsion I

| COMPOUND/MIXTURE | % (weight/weight) |
|---|---|
| - Urea | 10% |
| Lipophilic emollient mixture: | 13,4% |
| - Isopropyl Myristate | |
| - Paraffinum Liquidum | |
| - Cetearyl Ethylhexanoate | |
| - Dimethicone | |
| - Persea Gratissima (Avocado) Oil | |
| Emulsifying mixture: | 7,7% |
| - Glyceryl Stearate | |
| - PEG-40 Stearate | |
| - Cetyl Alcohol | |
| - Stearic Acid | |
| - Sodium Carbomer | |
| Humectant/skin conditioning agent/pH adjuster mixture: | 4,205% |
| - Sorbitol | |
| - Allantoin | |
| - Lactic Acid | |
| Preservative mixture: | 0,27% |
| - Propylparaben | |
| - Methylparaben | |
| - 2-Bromo-2-Nitropropane-1,3-diol | |
| Fragance (Perfume): | 0,1% |
| + Water | up to 100% |

### Emulsion II

| COMPOUND/MIXTURE | % (weight/weight) |
|---|---|
| - Urea | 20% |
| Lipophilic emollient mixture: | 13,4% |
| - Isopropyl Myristate | |
| - Paraffinum Liquidum | |
| - Cetearyl Ethylhexanoate | |
| - Dimethicone | |
| - Persea Gratissima (Avocado) Oil | |
| Emulsifying mixture: | 10,3% |
| - Glyceryl Stearate | |
| - PEG-40 Stearate | |
| - Cetyl Alcohol | |
| - Stearic Acid | |
| - Sodium Carbomer | |
| - Acrylates/C10-30 Alkyl Acrylate Crosspolymer | |
| Humectant/skin conditioning agent/pH adjuster mixture: | 4,206% |
| - Sorbitol | |
| - Allantoin | |
| - Lactic Acid | |
| Preservative mixture: | 0,27% |
| - Propylparaben | |
| - Methylparaben | |
| - 2-Bromo-2-Nitropropane-1,3-diol | |
| Fragance (Perfume): | 0,1% |
| + Water | up to 100% |

### Emulsion III

| COMPOUND/MIXTURE | % (weight/weight) |
|---|---|
| - Urea | --- |
| Lipophilic emollient mixture: | 13,4% |
| - Isopropyl Myristate | |
| - Paraffinum Liquidum | |
| - Cetearyl Ethylhexanoate | |
| - Dimethicone | |
| - Persea Gratissima (Avocado) Oil | |
| Emulsifying mixture: | 9,0% |
| - Glyceryl Stearate | |
| - PEG-40 Stearate | |
| - Cetyl Alcohol | |
| - Stearic Acid | |
| - Sodium Carbomer | |
| - Acrylates/C10-30 Alkyl Acrylate Crosspolymer | |
| Humectant/skin conditioning agent/pH adjuster mixture: | 4,205% |
| - Sorbitol | |
| - Allantoin | |
| - Lactic Acid | |
| Preservative mixture: | 0,27% |
| - Propylparaben | |
| - Methylparaben | |
| - 2-Bromo-2-Nitropropane-1,3-diol | |
| Fragance (Perfume): | 0,1% |
| + Water | up to 100% |

### Study

### 1. Treatment

Tests were conducted with human volunteers. The age ranged from 21 to 59 years. All were non-smokers and had no history of any severe skin disease.
The emulsions I, II, respectively III were applied to the skin of the volunteers as following:
All volunteers were treated once daily for 4 weeks with each of the emulsions I, II and III at three different areas of buttock skin. Skin biopsies from the three areas were taken thereafter to test the gene expression as described thereafter.

### 2. Assessment of gene expression

For assessment of gene expression total RNA was extracted from frozen biopsies (∅ 4 mm) and gene expression measured by semiquantitative RT-PCR. For isolation of RNA from frozen skin biopsies, 600 µl lysis buffer from PeqGold Total RNA Kit (PeqLab, Erlangen, Germany) were added and the samples were disrupted in a MixerMill MM300 (Retsch, Haan, Germany) three times for 3 min with 30 Hz. 50 ng total RNA were used for cDNA synthesis. PCR reactions were performed in an Opticon 1 (MJ Research, Waltham, MA, USA) using Sybr QPCR Supermix w. Rox (Invitrogen, Karlsruhe, Germany). PCR conditions were as follows: activation of hot start taq polymerase 94° C 15 minutes; denaturation 95° C, 20 seconds; annealing, 55° C, 20 seconds; extension, 72° C 30 seconds. Each sample was subjected to PCR in double using the appropriate primer pairs for 45-50 cycles. For comparison of relative expression in real time PCR control cells and treated cells the 2^{(-delta delta C(T))} method was used (Livak KJ, and Schmittgen TD. Analysis of relative gene expression data using real time quantitative PCR and the 2 (-delta delta C(T)) method. Methods 2001: 25: 402 - 408). mRNA expression of the genes of interest was shown as fold induction as compared to untreated skin samples of the same volunteer. Primers used are listed in Table 1.

**Table 1: Primer pairs for real time PCR**

| Gene | Primer pairs |
|---|---|
| 18S rRNA | 5'-GCCGCTAGAGGTGAAATTCTTG-3' |
| | 5'-CATTCTTGGCAAATGCTTTCG'-3' |
| Transglutaminase-1 | 5'-CCCCCGCAATGAGATCTACA-3' |
| | 5'-ATCCTCATGGTCCACGTACACA-3' |
| involucrin | 5'-CCCATCAGGAGCAAATGAAAC-3' |
| | 5'-GCTCGACAGGCACCTTCTG-3' |
| filaggrin | 5'-AAGGAACTTCTGGAAAAGGAATTTC-3' |
| | 5'-TTGTGGTCTATATCCAAGTGATCCAT-3' |
| loricrin | 5'-TCACATTGCCAGCATCTTCTCT-3' |
| | 5'-GGCTGCTTTTTCTGATAAGACATCT-3' |

For statistical analysis, the experimental data were analyzed using the Wilcoxon signed-rank test.

### 3. Assessment of elasticity

The elasticity of the skin was determined by applying tension (traction) to regions of the skin and measuring the velocity of moving back. An exemplary assay for quantifying the elasticity is based on the Digital Image Speckle Correlation (see e.g. Guan et al. (2004), "Determining Mechanical Properties of Rat Skin With Digital Image Speckle Correlation". Dermatology Vol. 208: 112-119).

### 4. Results

As shown in Table 2, treatment with emulsion I as well as treatment with emulsion II resulted in a significant increase of expression of the genes studied herein as compared to treatment with emulsion III and as compared to the control.
A detailed set of data is given in Table 3.

**Table 2: Level of induction of gene expression by topical application of emulsions I, II and III.**

| Gene | Emulsion | % of urea in emulsion | x-fold Induction +/- standard deviation |
|---|---|---|---|
| transglutaminase-1 | Emulsion III | 0% | 4.8 +/-6.8 |
| transglutaminase-1 | Emulsion I | 10% | 7.4 +/- 9.6 |
| transglutaminase-1 | Emulsion II | 20% | 7.8 +/- 11.3 |
| involucrin | Emulsion III | 0% | 5.0 +/- 6.2 |
| involucrin | Emulsion I | 10% | 7.0 +/- 8.1 |
| involucrin | Emulsion II | 20% | 6.7 +/- 9.1 |
| filaggrin | Emulsion III | 0% | 3.8 +/- 3.2 |
| filaggrin | Emulsion I | 10% | 5.3 +/- 7.4 |
| filaggrin | Emulsion II | 20% | 5.0 +/- 5.9 |
| loricrin | Emulsion III | 0% | 2.7 +/- 1.9 |
| loricrin | Emulsion I | 10% | 6.3 +/- 12.6 |
| loricrin | Emulsion II | 20% | 5.8 +/- 9.2 |

**Table 3: Level of induction of gene expression in the individual volunteers**

| | Emulsion III (0% urea) | | | | Emulsion I (10% urea) | | | | Emulsion II (20% urea) | | | |
|---|---|---|---|---|---|---|---|---|---|---|---|---|
| Vol. | TG | INV | LOR | FIL | TG | INV | LOR | FIL | TG | INV | LOR | FIL |
| 1 | 2.6 | 1.4 | 2.5 | 2.8 | 2.2 | 1.9 | 1.7 | 2.7 | 2.4 | 3.0 | 3.7 | 4.2 |
| 2 | 1.9 | 1.7 | 8.0 | 3.0 | 1.9 | 2.4 | 2.4 | 4.0 | 8.2 | 6.0 | 3.6 | 5.5 |
| 3 | 28.2 | 19.2 | 1.3 | 2.0 | 1.1 | 0.9 | 3.9 | 1.5 | 37.4 | 29.8 | 1.1 | 1.6 |
| 4 | 1.1 | 0.9 | 4.3 | 1.9 | 1.6 | 1.5 | 1.2 | 2.1 | 1.5 | 1.0 | 1.5 | 2.9 |
| 5 | 1.7 | 1.8 | 2.2 | 3.5 | 3.9 | 4.3 | 1.1. | 5.8 | 2.6 | 3.0 | 1.1 | 4.1 |
| 6 | 1.7 | 1.2 | 1.3 | 1.6 | 1.4 | 1.5 | 1.7 | 2.0 | 3.5 | 3.4 | 4.5 | 4.8 |
| 7 | 2.8 | 20.7 | 1.8 | 14.8 | 28.2 | 26.1 | 1.5 | 4.8 | 3.0 | 2.2 | 1.4 | 1.3 |
| 8 | 1.0 | 1.2 | 2.3 | 1.6 | 1.4 | 2.0 | 4.5 | 1.9 | 1.2 | 1.0 | 2.5 | 1.5 |
| 9 | 1.5 | 2.2 | 4.6 | 9.0 | 14.8 | 10.7 | 8.0 | 13.1 | 5.5 | 4.4 | 2.5 | 4.3 |
| 10 | 1.8 | 2.5 | 1.8 | 1.7 | 11.0 | 9.6 | 2.8 | 4.6 | 6.2 | 4.0 | 2.7 | 1.2 |
| 11 | 18.5 | 13.7 | 1.9 | 1.8 | 6.9 | 9.3 | 1.8 | 1.5 | 17.3 | 15.7 | 3.4 | 3.4 |
| 12 | 4.6 | 3.7 | 0.5 | 1.1 | 3.8 | 3.5 | 1.7 | 1.4 | 1.6 | 1.2 | 1.2 | 0.5 |
| 13 | 3.0 | 3.1 | 1.9 | 2.0 | 1.6 | 1.7 | 1.8 | 2.2 | 1.5 | 0.9 | 1.0 | 1.2 |
| 14 | 0.9 | 1.0 | 1.2 | 1.8 | 1.5 | 1.9 | 3.5 | 3.7 | 1.0 | 1.0 | 1.4 | 1.6 |
| 15 | 1.2 | 1.1 | 1.5 | 2.1 | 1.1 | 1.3 | 3.2 | 3.7 | 1.4 | 1.1 | 1.3 | 1.4 |
| 16 | 7.3 | 10.0 | 1.6 | 3.4 | 30.8 | 27.8 | 8.8 | 5.9 | 9.1 | 11.2 | 5.0 | 3.2 |
| 17 | 1.6 | 1.6 | 6.1 | 4.9 | 3.2 | 3.3 | 9.1 | 6.4 | 3.9 | 3.0 | 35.6 | 15.5 |
| 18 | 5.3 | 3.4 | 3.9 | 3.5 | 6.3 | 14.3 | 60.1 | 35.6 | 8.4 | 11.4 | 28.0 | 25.9 |
| 19 | 1.2 | 0.9 | 1.4 | 1.7 | 1.4 | 1.6 | 2.2 | 3.4 | 1.4 | 1.6 | 2.7 | 4.1 |
| 20 | 2.0 | 2.0 | 4.3 | 3.1 | 3.8 | 3.7 | 9.3 | 4.2 | 4.8 | 3.7 | 14.4 | 10.4 |
| 21 | 10.6 | 11.1 | 2.0 | 1.3 | 26.8 | 17.9 | 2.7 | 1.5 | 41.3 | 32.8 | 2.1 | 5.5 |

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A use of a composition comprising urea for increasing the epidermal expression of at least one gene selected from the group comprising transglutaminase-1, involucrin, filaggrin and loricrin.

2. A use according to claim 1 wherein said composition comprises more than 5% by weight, based on the total amount of the composition, of urea.

3. A use according to claim 1 or 2 wherein the expression of any of said genes is increased by a factor of at least 3, preferably at least 5, more preferably at least 7, even more preferably at least 9, most preferably at least 12.

4. A use according to any one of claims 1 or 3 wherein said composition comprises at most 30% by weight, preferably 10 to 25% by weight of urea.

5. A use according to any one of claims 1 to 4 wherein said composition comprises at least 50% by weight, based on the total amount of the composition, of hydrophobic components.

6. A use according to any one of claims 1 to 5 wherein said composition is a cosmetic composition, preferable further comprising at least one compound selected from the group comprising UV-filters, vitamins, coenzymes, retinoids, phytokines and antioxidative agents.

7. A use according to claim 6 wherein said UV-filter is at least one UV-filter selected from the group comprising pyrroltriazines, benzotriazolylphenoles and heptaazaphenalenes.

8. A use according to any one of claims 1 to 5 wherein said composition is a pharmaceutical composition, preferable further comprising at least one pharmaceutically active agent.

9. A use according to any one of claims 1 to 8 wherein said composition is applied topically onto the skin or administered by injection.

10. A use according to claim 9 wherein said composition is applied at least once a day for at least 2 weeks topically and/or administered by injection and optionally longer until the expression of any of said genes is increased by a factor of at least 3, preferably at least 5, more preferably at least 7, even more preferably at least 9, most preferably at least 12.

11. A use according to claim 10 for preventing a decrease of the elasticity of the skin or enhancing said elasticity.

12. A use according to claim 10 for preventing and/or treating damages of the skin associated with a decrease of elasticity.

13. A use according to claim 11 or 12 wherein said damages are associated with aging and/or exposure to at least one environmental toxins, UV-light, dermatoses and/or infections of the skin.
